(19) Europäisches Patentamt
European Patent Office
Office européen des brevets

(11) Publication number: **0 094 769**
**B1**

(12) **EUROPEAN PATENT SPECIFICATION**

(45) Date of publication of patent specification: **26.11.86**

(21) Application number: **83302551.3**

(22) Date of filing: **05.05.83**

(51) Int. Cl.⁴: **C 07 D 307/86,** C 07 D 493/04 // A61K31/35 ,(C07D493/04, 311:00, 309:00)

(54) **Preparing 7-alkoxybenzofurans and intermediates used therein.**

(30) Priority: **17.05.82 US 378686**

(43) Date of publication of application: **23.11.83 Bulletin 83/47**

(45) Publication of the grant of the patent: **26.11.86 Bulletin 86/48**

(84) Designated Contracting States: **BE CH DE FR GB IT LI NL SE**

(56) References cited:
**FR-A-2 387 227**
**US-A-2 680 119**
**US-A-4 284 569**

(73) Proprietor: **THE UPJOHN COMPANY**
**301 Henrietta Street**
**Kalamazoo, Michigan 49001 (US)**

(72) Inventor: **Gammill, Ronald Bruce**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 094 769 B1

**Description**

The present invention relates to 7-alkoxybenzofurans and to their preparation. Such compounds are of value as intermediates in the preparation of khellin and analogues thereof. Khellin and related compounds are known to exert a wide variety of pharmacological effects, including useful anti-atheroschlerotic activity.

US—A—2680119 describes a synthesis of khellin and related compounds, from dimethoxyresorcinol. An intermediate in the synthesis is 1-(6-hydroxy-4,7-dimethoxy-5-benzofuranyl)ethanone, i.e. a compound of formula XVII in which $R_2$ and $R_3$ are each methyl. This compound is also disclosed in FR—A—2387227.

US—A—4284569 discloses 1-(6-hydroxy-7-methoxy-5-benzofuranyl)ethanone, i.e. a compound of formula XVI wherein $R_2$ is methyl, as being useful in the preparation of a wide variety of anti-atherosclerotic substances including khellin and various analogues thereof.

According to a first aspect of the present invention, a process for preparing a dialkoxybenzofuran of formula XVII, wherein $R_2$ and $R_3$ are the same or different $C_{1-4}$ alkyl groups, comprises reacting an alkoxybenzofuran of formula XVI, wherein $R_2$ is $C_{1-4}$ alkyl, with an oxidising reagent selected from thallium (III) nitrate, ceric ammonium nitrate and lead tetraacetate, in a $C_{1-4}$ alkanol solvent.

Novel dialkoxybenzofurans of formula XVII are those wherein $R_2$ and $R_3$ are different.

Novel alkoxybenzofurans of formula XVI are those wherein $R_2$ is $C_{2-4}$ alkyl.

Further novel compounds according to the present invention are alkoxydihydrobenzofurans of formula XV, wherein $R_2$ is $C_{1-4}$ alkyl. Such compounds are prepared by a process which comprises:

(a) converting pyrogallol to 3,6,7-benzofurantriol triacetate;

(b) reducing and deacetoxylating the triacetate to give 2,3-dihydro-6,7-benzofurandiol diacetate;

(c) subjecting the diacetate to a Fries rearrangement, to give 1-(2,3-dihydro-6,7-dihydroxy-5-benzofuranyl)ethanone; and

(d) selectively $C_{1-4}$ alkoxylating the ketone.

The compounds of formula XVI can be converted to 4,9-dialkoxyfurochromones by the procedure described in US—A—4284569 (or by analogy to that procedure when $R_2$ is other than methyl).

In step (a) as defined above, pyrogallol is converted to the triacetate by treatment with chloroacetonitrile, according to procedures described by Geissman *et al*, J.A.C.S. *73* (1951) 57—65.

In step (b), the diacetate is obtained from the triacetate, using a metal catalyst under a hydrogen atmosphere. For example, a conventional metal catalyst such as palladium and carbon catalyst is used. See Dann and Zeller, Ber. *93* (1960) 28—29.

In step (c), the diacetate is subjected to Fries rearrangement to yield the ketone, by treatment with a mixture of aluminium trichloride and nitrobenzene.

In step (d), the ketone is selectively alkylated to the formula XV compound, by treatment with a $C_{1-4}$ alkyl iodide. Any concomitant desalkoxylation in this reaction is reversed by treatment with hydrobromic acid.

A formula XV compound is dehydrogenated, using 2,3-dichloro-5,6-dicyanobenzoquinone (DDQ), to give a formula XVI compound. See Linn *et al*, J. Heterocyclic Chem. (1979) 799.

A formula XVII compound is produced by alkoxylation, e.g. methylation, of a formula XVI compound. The alkoxylation employs an oxidising agent in a $C_{1-4}$ alkanol solvent corresponding to the alkoxy group to be introduced at C-4. $Ce(NH_4)_2(NO_3)_6$ or $Pb(OAc)_4$ may be employed as the oxidising agent; see Brother *et al*, Helv. Chim. Acta. *35* (1952) 9—10 (1952). Alternatively, thallium (III) nitrate is employed as the oxidising agent; see Taylor, J. Org. Chem. *41* (1976) 282. In the latter case, maximum yields are obtained when the oxidising agent is added over a period of about 15 min, the reaction mixture being maintained at about −25°C for 30 min, followed by heating for 1 to 2 min.

An alternative synthesis of formula XVII compounds in which $R_2$ and $R_3$ are the same is described and claimed in EP—B—95.835. An example of their conversion to 4,9-diethoxy-7-methylthiomethylfurochromone is given.

The following Examples illustrate the invention.

## Example 1

3,6,7-Benzofurantriol triacetate (Formula XII).

Refer to Chart A.

Dry zinc chloride (38 g) is added to pyrogallol (Formula XI, 35 g). To these solids under a nitrogen atmosphere are added diethyl ether and chloroacetonitrile (18 ml). The resulting mixture is then stirred and cooled to 0°C. Hydrochloride gas is then bubbled to the reaction mixture for 30 min, the mixture is allowed to warm slowly to ambient temperature with stirring for 12 hr. Thereafter, the two-face mixture is cooled to 0°C and the ether layer decanted. Additional diethyl ether (100 mg) is added, stirred, and decanted. Water (250 ml) is added to the resulting residue and the aqueous mixture is then refluxed for 30 min yielding a homogeneous solution. The solution is then cooled to 4°C, filtered, and yields a reddish-brown solid (24.5 g), α-chlorogallacetophenone. Without further purification, the solid is then dissolved in ethanol (300 ml) containing sodium acetate (24.5 g). After refluxing for 5 hr, the resulting mixture is then dried and treated with acetic anhydride (150 ml) and pyridine (75 ml). The resulting mixture is then stirred at ambient temperature for 12 hr, decanted into ice water (700 ml) and stirred for one hr. The resulting precipitate is then collected on a filter, washed with water and air-dried. Thereafter, the filtrate is acidified with

concentrated hydrochloric acid and extracted with ethyl acetate. The organic extracts are then washed thoroughly with saturated sodium bicarbonated brine, dried over magnesium sulphate, and concentrated to a rsidue. Chromatography on 1.3 kg of silica gel, eluting with 40% ethyl acetate Skellysolve B ethyl acetate, gives 27.75 g of pure product, a white solid, melting point 99—101°C.

Example 2

2,3-Dihydro-6,7-benzofurandiol diacetate (Formula XIII).

A mixture of the title product of Example I (50 g) in ethyl acetate (350 ml) is treated with anhydrous potassium acetate (7.5 g) and 7.5 g of a 10% palladium-on-carbon catalyst. The reaction mixture is then hydrogenated at 65°C for 2 hr, cooled to ambient temperature, and filtered through diatomaceous earth. The resulting filtrate is then concentrated under reduced pressure, yielding a solid. Recrystallization from 250 ml of ethyl acetate and Skellysolve B (1:1) yields 33.65 g of pure title product as a white solid, melting point 114—115°C. Silica gel TLC $R_f$ is 0.31 in 25% ethyl acetate in hexane. IR absorptions ($cm^{-1}$) are observed at 1765, 1625, 1610, 1490, 1465, 1375, 1225, 1210, 1180, and 1035. NMR-absorptions are observed at 7.05, 6.62, 4.64, 3.21, 2.25, and 2.23 δ.

Example 3

1-(2,3-dihydro-6,7-dihydroxy-5-benzofuranyl)-ethanone (Formula XIV).

Refer to Chart A.

A mixture of the title product of Example 2 (9.50 g), nitrobenzene (100 ml) and aluminium trichloride (6.36 g) is heated at 60°C for 90 min. After cooling to ambient temperature, the reaction mixture is poured over ice and 2 N hydrochloric acid (100 ml) is added, followed by the addition of water (300 ml). After stirring for 3 hr, the reaction mixture is then extracted with ethyl acetate. The organic layer is separated and washed with 5% aqueous sodium hydroxide, and the aqueous layer then poured into 2 N hydrochloric acid (500 ml), yielding a precipitate. Ethyl acetate is then added and then separated from the aqueous layer. The organic layer is then dried over magnesium sulphate and concentrated under reduced pressure to yield 6.35 g of title product as a brown solid. Recrystallization from ethyl acetate yields pure crystalline product, melting point 190—190.5°C. Silica gel TLC $R_f$ is 0.25 in 5% ethyl acetate in trichloromethane. IR absorptions ($cm^{-1}$) are observed at 3460, 3200, 1645, 1605, 1490, 1445, 1365, 1320, 1255, and 1055. NMR absorptions are observed at 7.15, 4.70, 3.18, and 2.52 δ.

Example 4

1-(2,3-dihydro-6-hydroxy-7-methoxy-5-benzofuranyl)ethanone (Formula XV: $R_2$ is methyl).

Refer to Chart A.

A mixture of the title product of Example 3 (5.9 g), potassium carbonate (12 g) and methyl iodide (25 g) is heated at reflux and acetone for 18 hr. After cooling to ambient temperature and removal of the potassium carbonate by filtration, the resulting mixture is then concentrated under reduced pressure to a yellow oil. The oil is then dissolved in trichloromethane and treated with hydrobromic acid and refluxed for 2 hr. After cooling to ambient temperature and concentration under reduced pressure, chromatography eluting with 5% ethyl acetate in trichloromethane yields 6.0 g of pure title product, melting point 95—97°C. Silica gel TLC $R_f$ is 0.5 in 5% ethyl acetate in trichloromethane. IR absorptions ($cm^{-1}$) are observed at 2700, 1630, 1615, 1430, 1405, 1365, 1330, 1290, and 1060. NMR absorptions are observed at 7.3, 6.48, 3.9, and 3.15 δ.

Example 5

1-(6-hydroxy-7-methoxy-5-benzofuranyl)-ethanone (Formula XVI: $R_2$ is methyl).

Refer to Chart A.

To a solution of the title product of Example 4 (12.1 g) in dioxane is added 2,3-dichloro-5,6-dicyanobenzoquinone (DDQ, 17.8 g). The resulting black solution is then stirred at reflux for 2 hr. Upon cooling to ambient temperature, a precipitate forms. The reaction mixture is then filtered and the filter cake is washed with dichloromethane. The filtrate is then concentrated to a residue and the residue chromatographed on 700 g of silica gel eluting with dichloromethane. Pure title product is obtained as 5.5 g of an oil which spontaneously crystallizes. Recrystallization from ethyl acetate in hexane (1:5) yields pure title product, melting point 61.2—63.5°C. Silica gel TLC $R_f$ is 0.31 in 25% ethyl acetate in hexane. IR absorptions ($cm^{-1}$) are observed at 3140, 3110, 2720, 1635, 1625, 1545, 1320, 1300, 1275, and 1050. NMR absorptions are observed at 7.78, 7.65, 4.21, and 2.71 δ.

Example 6

1-(6-hydroxy-4,7-dimethoxy-5-benzofuranyl)-ethanone (Formula XVII: $R_2$ and $R_3$ are both methyl).

Refer to Chart A.

A. The title product of Example 5 (100 mg) is added to methanol (4 ml) and cooled to −25°C. To the resulting heterogeneous mixture is added a methanolic (7 ml) solution of thallium (III) nitrate trihydrate, $Tl(ONO_2)_3 \cdot 3H_2O$ (250 mg), dropwise over about 15 min. The resulting mixture is then stirred for 30 min at −25°C and heated to reflux for 1—2 min. The reaction is then poured into saturated aqueous sodium bicarbonate and extracted with diethyl ether. The etheral layer is then dried over magnesium sulphate and

concentrated under reduced pressure to yield a yellow oil. Crystallization is achieved by dissolving the oil in 1% ethyl acetate in hexane and cooling to 0°C for 12 hr. Filtration of the resulting crystals yields 70 mg of pure title product, melting point 98—99°C. Silica gel TLC $R_f$ is 0.6 in ethyl acetate and hexane (1:1). IR absorptions ($cm^{-1}$) are observed at 2955, 2930, 2926, 2868, 1629, 1619, 1587, 1471, 1452, 1444, 1425, 1382, 1364, 1303, 1267, 1151, 1079, 1061, and 755. NMR absorptions are observed at 7.5, 6.9, 4.15, 4.05, and 2.7 δ.

B. Alternatively, title product is prepared utilizing lead tetraacetate (200 mg) which is added to methanol (6 ml) and cooled to 0°C. To the resulting solution is added the title product of Example 5 (100 mg) dropwise in methanol (5 ml). The resulting mixture is then stirred at 0°C for 80 min and poured into saturated aqueous sodium bicarbonate. After extraction with ether, the etheral solution is then dried over magnesium sulphate and concentrated under reduced pressure to yield a yellow oil. This crude solid is then dissolved in methanol and heated at reflux for 1 hr. After cooling to ambient temperature and removal of solvent under reduced pressure, crystallization from 1% ethyl acetate in hexene yields 70 mg of pure title product, melting point 98—100°C.

Example 7

1-(2,3-dihydro-6-hydroxy-7-ethoxy-5-benzofuranyl)ethanone (Formula XV: $R_2$ is ethyl)
Refer to Chart A.

A mixture of the title product of Example 3 (5.9 g), potassium carbonate (12 g) and ethyl iodide (28 g) is heated at reflux and acetone for 18 hr. After cooling to ambient temperature and removal of the potassium carbonate by filtration, the resulting mixture is then concentrated under reduced pressure. The residue is then dissolved in trichloromethane and treated with hydrobromic acid and refluxed for 2 hr. After cooling to ambient temperature and concentration under reduced pressure, chromatography, eluting with 5% ethyl acetate in trichloromethane, yields 6.0 g of formula XV product.

Example 8

1-(6-hydroxy-7-ethoxy-5-benzofuranyl)ethanone (Formula XVI: $R_2$ is ethyl)
Refer to Chart A.

To a solution of the product of Example 7 (12 g) in dioxane is added 2,3-dichloro-5,6-dicyanobenzoquinone (DDQ, 17.8 g). The resulting black solution is then stirred at reflux for 2 hr. Upon cooling to ambient temperature, a precipitate forms. The reaction mixture is then filtered and the filter cake is washed with dichloromethane. The filtrate is then concentrated to a residue and the residue chromatographed on 700 g of silica gel, eluting with dichloromethane, to obtain title product.

Example 9

1-(6-hydroxy-4-methoxy-7-ethoxy-5-benzofuranyl)ethanone (Formula XVII: $R_2$ is ethyl and $R_3$ is methyl)
Refer to Chart A.

The formula XVI product of Example 8 (100 mg) is added to methanol (4 ml) and cooled to −25°C. To the resulting mixture is added a methanolic (7 ml) solution of thallium (III) nitrate trihydrate, $Tl(ONO_2)_3.3H_2O$ (250 mg), dropwise over about 15 min. The resulting mixture is then stirred for 30 min at −25°C and heated to reflux for 1—2 min. The reaction is then poured into saturated aqueous sodium bicarbonate and extracted with diethyl ether. The ethereal layer is then dried over magnesium sulphate and concentrated under reduced pressure to yield formula XVII product.

Example 10

1-(6-hydroxy-4-ethoxy-7-methoxy-5-benzofuranyl)ethanone (Formula XVII: $R_2$ is methyl and $R_3$ is ethyl)
Refer to Chart A.

The title product of Example 5 (100 mg) is added to ethanol (4.5 ml) and cooled to −25°C. To the resulting mixture is added an ethanolic (8 ml) solution of thallium (III) nitrate trihydrate, $Tl(ONO_2)_3.3H_2O$ (250 mg), dropwise over about 15 min. The resulting mixture is then stirred for 30 min at −25°C and heated to reflux for 1—2 min. The reaction mixture is then poured into saturated aqueous sodium bicarbonate and extracted with diethyl ether. The ethereal layer is then dried over magnesium sulphate and concentrated under reduced pressure to yield formula XVII product.

CHART A

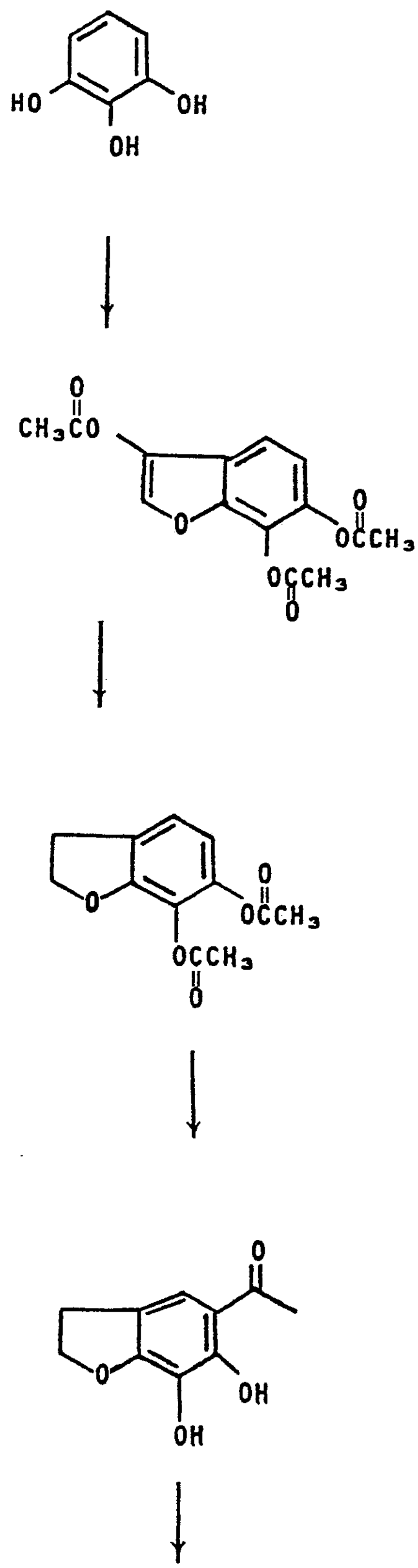

XI

XII

XIII

XIV

CHART A (cont'd)

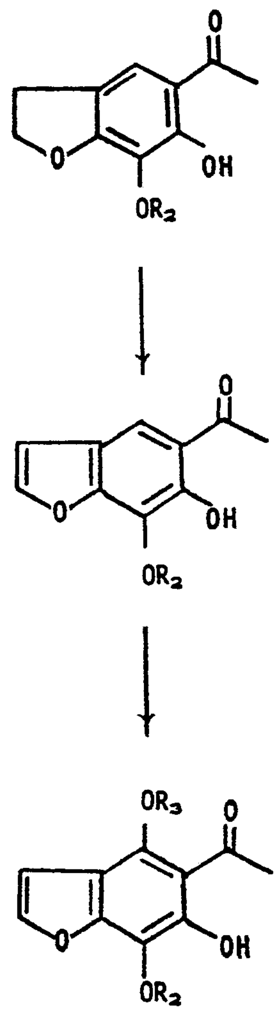

XV

XVI

XVII

**Claims**

1. A process for preparing a dialkoxybenzofuran of the formula

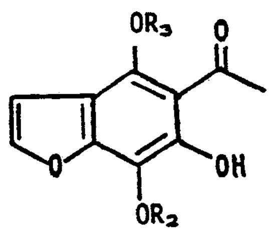

XVII

wherein $R_2$ and $R_3$ are the same or different $C_{1-4}$ alkyl groups, which comprises reacting an alkoxybenzofuran of the formula

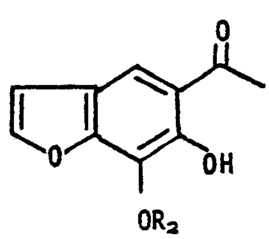

XVI

wherein $R_2$ is $C_{1-4}$ alkyl, with an oxidising reagent selected from thallium (III) nitrate, ceric ammonium nitrate and lead tetraacetate, in a $C_{1-4}$ alkanol solvent.

2. A dialkoxybenzofuran of formula XVII as defined in claim 1, wherein $R_2$ and $R_3$ are different.

3. An alkoxybenzofuran of formula XVI as defined in claim 1, wherein $R_2$ is $C_{2-4}$ alkyl.

4. An alkoxydihydrobenzofuran of the formula

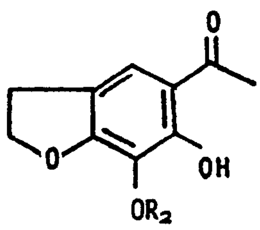

XV

wherein $R_2$ is $C_{1-4}$ alkyl.

5. A process for preparing an alkoxydihydrobenzofuran as claimed in claim 4, which comprises:

(a) converting pyrogallol to 3,6,7-benzofurantriol triacetate (XII);

(b) reducing and deacetoxylating the triacetate to give 2,3-dihydro-6,7-benzofurandiol diacetate (XIII);

(c) subjecting the diacetate to a Fries rearrangement, to give 1-(2,3-dihydro-6,7-dihydroxy-5-benzofuranyl)ethanone (XIV); and

(d) selectively $C_{1-4}$ alkoxylating the ketone.

**Patentansprüche**

1. Ein Verfahren zur Bereitung eines Dialkoxybenzofurans der Formel

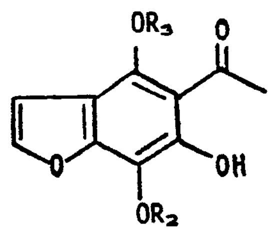

XVII

wobei $R_2$ und $R_3$ dieselben oder verschiedene $C_{1-4}$Alkylgruppen sind, bei dem ein Alkoxybenzofuran der Formel

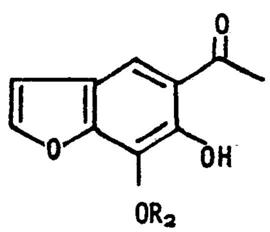

XVI

wobei $R_2$ ein $C_{1-4}$Alkyl ist, mit einem aus Thallium (III) Nitrat, Zerammoniumnitrat und Bleitetraacetat gewählten Oxydierungsreagens in einem $C_{1-4}$ Alkanollösungsmittel zur Reaktion gebracht wird.

2. Ein Dialkoxybenzofuran der Formel XVII wie in Anspruch 1 definiert, wobei $R_2$ und $R_3$ verschieden sind.

3. Ein Alkoxybenzofuran der Formel XVI wie in Anspruch 1 definiert, wobei $R_2$ $C_{2-4}$Alkyl ist.

4. Ein Alkoxydihydrobenzofuran der Formel

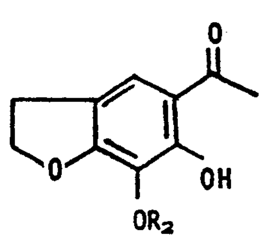

XV

wobei $R_2$ $C_{1-4}$Alkyl ist.

5. Ein verfahren zur Bereitung eines Alkoxydihydrobenzofurans wie in Anspruch 4, bei welchem
(a) Pyrogallol in 3,6,7-Benzofurantrioltriacetat (XII) überführt wird;
(b) das Triacetat zu 2,3-Dihydro-6,7-Benzofurandiol Diacetat (XIII) reduziert und deacetoxyliert wird;
(c) das Diacetat einer Fries-Umlagerung unterzogen wird, um 1-(2,3-Dihydro-6,7-Dihydroxy-5-Benzofuranyl)Äthanon (XIV) zu ergeben; und
(d) das Keton selektiv $C_{1-4}$ alkoxyliert wird.

**Revendications**

1. Procédé de préparation d'un dialkoxybenzofuranne de formule

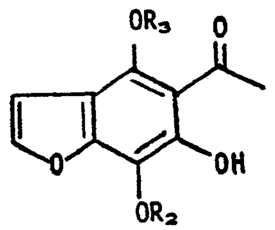

XVII

dans laquelle $R_2$ et $R_3$ sont des groupes alkyle en $C_1$ à $C_4$ identiques ou différents, qui consiste à faire réagir un alkoxybenzofuranne de formule

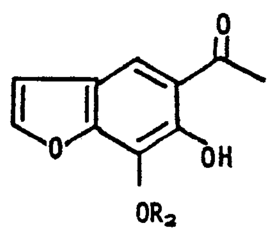

XVI

dans laquelle $R_2$ est un groupe alkyle en $C_1$ à $C_4$, avec un réactif oxydant choisi entre le nitrate de thallium (III), le nitrate cérique d'ammonium et le tétraacétate de plomb dans un solvant alcanolique en $C_1$ à $C_4$.

2. Dialkoxybenzofuranne de formula XVII suivant la revendication 1, dans lequel $R_2$ et $R_3$ sont différents.

3. Alkoxybenzofuranne de formule XVI suivant la revendication 1, dans lequel $R_2$ est un groupe alkyle en $C_2$ à $C_4$.

4. Alkoxydihydrobenzofuranne de formule

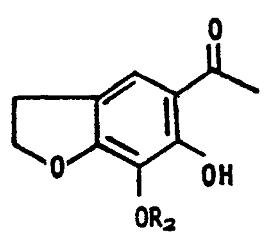

XV

dans laquelle $R_2$ est un groupe alkyle en $C_1$ à $C_4$.

5. Procédé de préparation d'un alkoxydihydrobenzofuranne suivant la revendication 4, qui consiste:
(a) à transformer du pyrogallol en triacétate de 3,6,7-benzofurannetriol (XII);
(b) à réduire et désacétoxyler le triacétate pour obtenir le diacétate de 2,3-dihydro-6,7-benzofurannediol (XIII);
(c) à soumettre le diacétate à une transposition de Fries pour obtenir la 1-(2,3-dihydro-6,7-dihydroxy-5-benzofurannyl)éthanone (XIV); et
(d) à alkoxyler sélectivement en $C_1$ à $C_4$ la cétone.